# EUROPÄISCHE PATENTSCHRIFT

(11) **EP 1 313 763 B1**
(45) Veröffentlichungstag und Bekanntmachung des Hinweises auf die Patenterteilung: **08.03.2006**
(21) Anmeldenummer: 01960288.7
(22) Anmeldetag: 08.06.2001
(51) Int. Cl.: C07K 14/47

(54) **VERFAHREN ZUR GEWINNUNG UND ANWENDUNG VON ANTIBIOTISCH WIRKSAMEN PEPTIDEN ZUR BEHANDLUNG VON INFEKTIONSKRANKHEITEN**
METHOD FOR THE EXTRACTION AND THE USE OF ANTIBIOTICALLY EFFECTIVE PEPTIDES FOR TREATING INFECTIOUS DISEASES
PROCEDE DE PREPARATION ET D'UTILISATION DE PEPTIDES A EFFICACITE ANTIBIOTIQUE POUR TRAITER DES MALADIES INFECTIEUSES

(30) Priorität: 08.06.2000 DE 10027992
(43) Veröffentlichungstag der Anmeldung: 28.05.2003
(60) Teilanmeldung: 04019254.4
(73) Patentinhaber: IPF Pharmaceuticals GmbH, 30625 Hannover (DE)
(72) Erfinder: LIEPKE, Cornelia, 30625 Hannover (DE); ARNDT, Susann, 30625 Hannover (DE); FORSSMANN, Wolf-Georg, 30625 Hannover (DE)
(74) Vertreter: Meyers, Hans-Wilhelm
(86) Internationale Anmeldenummer: PCT/EP2001/006518
(87) Internationale Veröffentlichungsnummer: WO 2001/094386

(56) Entgegenhaltungen:
- WO-A-98/31706
- TERZI E ET AL: "ISOLATION AND AMINO ACID SEQUENCE OF A NOVEL 6.8-KDA MITOCHONDRIAL PROTEOLIPID FROM BEEF HEART USE OF FAB-MS FOR MOLECULAR MASS DETERMINATION" FEBS (FEDERATION OF EUROPEAN BIOCHEMICAL SOCIETIES) LETTERS, Bd. 260, Nr. 1, 1990, Seiten 122-126, XP001071177 ISSN: 0014-5793 -& DATABASE EBI [Online] SWALL; 1. April 1990 (1990-04-01) TERZI, E. ET AL.: "6.8 kDa mitochondrial proteolipid" Database accession no. P14790 XP002199874
- MIYAKAWA Y ET AL: "IN VITRO ACTIVITY OF THE ANTIMICROBIAL PEPTIDES HUMAN AND RABBIT DEFENSINS AND PORCINE LEUKOCYTE PROTEGRIN AGAINST MYCOBACTERIUM TUBERCULOSIS" INFECTION AND IMMUNITY, AMERICAN SOCIETY FOR MICROBIOLOGY. WASHINGTON, US, Bd. 64, Nr. 3, März 1996 (1996-03), Seiten 926-932, XP002920633 ISSN: 0019-9567 -& WO 98 51794 A (HUMAN GENOME SCIENCES, INC.) 19. November 1998 (1998-11-19)
- PIERS K L ET AL: "RECOMBINANT DNA PROCEDURES FOR PRODUCING SMALL ANTIMICROBIAL CATIONIC PEPTIDES IN BACTERIA" GENE, ELSEVIER BIOMEDICAL PRESS. AMSTERDAM, NL, Bd. 134, 1993, Seiten 7-13, XP002034848 ISSN: 0378-1119
- HANCOCK R E W ET AL: "ANTIMICROBIAL PEPTIDES: BROAD-SPECTRUM ANTIBIOTICS FROM NATURE" CLINICAL MICROBIOLOGY AND INFECTION, XX, XX, Bd. 1, Nr. 4, 1. Juni 1996 (1996-06-01), Seiten 226-229, XP000616655 ISSN: 1198-743X
- TANG Y Q ET AL: "A CYCLIC ANTIMICROBIAL PEPTIDE PRODUCED IN PRIMATE LEUKOCYTES BY THE LIGATION OF TWO TRUNCATED ALPHA-DEFENSINS" SCIENCE, AMERICAN ASSOCIATION FOR THE ADVANCEMENT OF SCIENCE,, US, Bd. 286, Nr. 5439, 1999, Seiten 498-502, XP000919300 ISSN: 0036-8075
- BALS ROBERT ET AL: "Mouse beta-defensin 3 is an inducible antimicrobial peptide expressed in the epithelia of multiple organs" INFECTION AND IMMUNITY, AMERICAN SOCIETY FOR MICROBIOLOGY. WASHINGTON, US, Bd. 67, Nr. 7, Juli 1999 (1999-07), Seiten 3542-3547, XP002160217 ISSN: 0019-9567
- MERLUZZI ET AL: "BIOLOGICAL ACTIVITY OF A NOVEL CATHELICIDIN-DERIVED ANTIMICROBIAL PEPTIDE FROM SHEEP" PEPTIDES, ELMSFORD, US, 1998, Seiten 1639-1640, XP002157166 ISSN: 0196-9781
- ABEL VAN R J ET AL: "SYNTHESIS AND CHARACTERIZATION OF INDOLICIDIN, A TRYPTOPHAN-RICH ANTIMICROBIAL PEPTIDE FROM BOVINE NEUTROPHILS" INTERNATIONAL JOURNAL OF PEPTIDE AND PROTEIN RESEARCH, MUNKSGAARD, COPENHAGEN, DK, Bd. 45, Nr. 5, 1. Mai 1995 (1995-05-01), Seiten 401-409, XP000650607 ISSN: 0367-8377
- CHARLET ET AL: "Isolation of several cysteine-rich antimicrobial peptides from the blood of a mollusc, Mytilus edulis" JOURNAL OF BIOLOGICAL CHEMISTRY, AMERICAN SOCIETY OF BIOLOGICAL CHEMISTS, BALTIMORE, MD, US, Bd. 271, Nr. 36, 6. September 1996 (1996-09-06), Seiten 21808-21813, XP002132658 ISSN: 0021-9258
- CAMMUE B P A ET AL: "GENE-ENCODED ANTIMICROBIAL PEPTIDES FROM PLANTS" CIBA FOUNDATION SYMPOSIUM, AMSTERDAM, NL, Bd. 186, 1994, Seiten 91-106, XP002032717 ISSN: 0300-5208

## Beschreibung

Die Erfindung betrifft antibiotische Peptide, Verfahren zu ihrer Herstellung sowie ihre Verwendung.

Es ist bekannt, dass natürlich vorkommende Peptide antibiotisch wirksam sein können. So beschreibt die WO 97/35877 das Vorkommen und die Aufreinigung von antibiotischen Peptiden aus Kuhmilch.

Überraschenderweise wurde nun gefunden, dass antibiotisch aktive Peptide auch aus humanem Plazentaextrakt und aus bovinem Thymusextrakt gewonnen werden können.

Dazu wurden die genannten Gewebeextrakte chromatographisch aufgereinigt, und die erhaltenen Fraktionen unter Anwendung des Radial-Diffusions-Hemmtests auf antimikrobielle Aktivität überprüft. Überraschenderweise wurde festgestellt, dass in den Fraktionen Substanzen enthalten sind, die das Wachstum von Bakterien stark hemmen. Durch weitere chromatographische Trennverfahren konnten die biologisch aktiven Substanzen konzentriert, zur Homogenität gereinigt und ihre Struktur aufgeklärt werden.

Die gereinigten Peptide wirken überraschenderweise sowohl auf Gram-positive Bakterien wie *Bacillus subtilis, Micrococcus luteus* und *Staphylococcus carnosus* als auch auf Gram-negative Bakterien wie *Escherichia coli* und *Pseudomonas fluorescens* stark wachstumshemmend. Die wachstumsmodulierende Eigenschaft der gereinigten Peptide auf Mikroorganismen wurde bei der Entwicklung des hier vorgestellten Verfahrens erstmals nachgewiesen.

Die vorliegende Erfindung betrifft folglich neue antimikrobiell wirksame Peptide, ihre Herstellung, diese Peptide enthaltende Arzneimittel sowie diese Peptide enthaltende Zubereitungen und ihre Verwendung.

Die vorliegende Erfindung betrifft ein Peptid mit der Aminosäuresequenz 5.

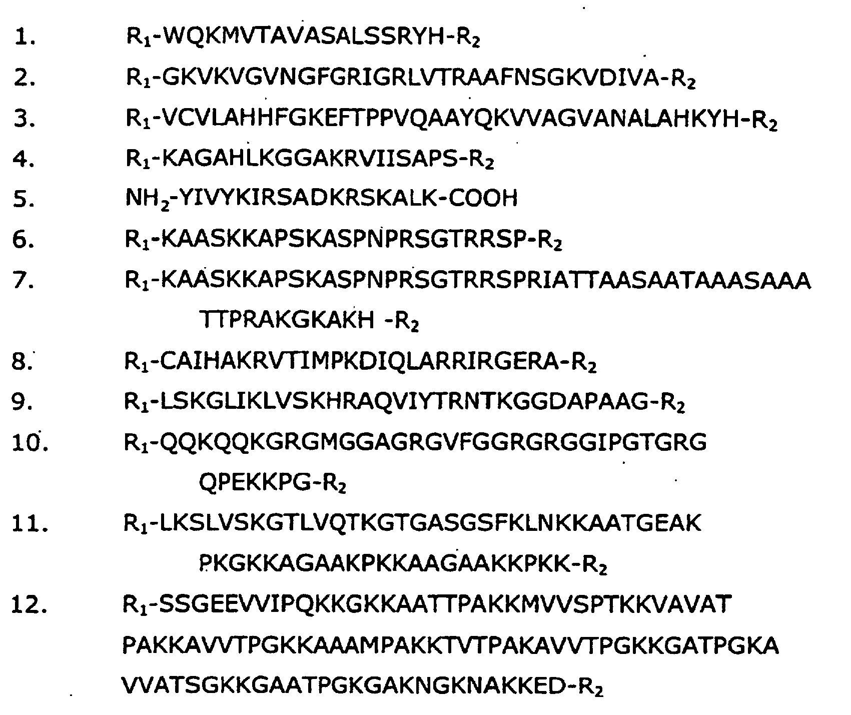

sowie die amidierten, acetylierten, sulfatierten, phosphorylierten, glycosylierten, oxydierten Derivate und weiterhin Fragmente mit antimikrobiellen Eigenschaften, die aus der Aminosäuresequenz abgeleitet werden, mit antimikrobielen Wirkung.

Die weiteren Peptide, der Aminosäuresequenzen 1. bis 4. und 6. bis 12., sind als Vergleichsbeispiele aufgeführt und nicht gegenstend der Erfindung. R₁ ist NH₂, R₂ ist COOH.

Neben den erwähnten Derivaten können die Peptide auch statt der natürlichen L-Aminosäuren D-Aminosäuren aufweisen, was den Abbau durch Proteasen verzögert.

Gegenstand der Erfindung sind auch Peptide, in denen einzelne Aminosäuren ausgetauscht sind, insbesondere durch sogenannte konservative Austausche innerhalb der bekannten Gruppen von sauren, basischen, neutralen, hydrophoben, aromatischen und hydrophilen Aminosäuren. Beispiele hierfür sind Serin gegen Alanin oder Alanin gegen Serin, Isoleucin gegen Leucin oder Leucin gegen Isoleucin usw.

Als Fragmente kommen insbesondere N- oder C-terminal verkürzte Peptide in Frage, aber auch Peptide, in denen einzelne Aminosäuren deletiert sind, aber nicht mehr als 10 % der Aminosäuren. Diese Fragmente sind insoweit Gegenstand der Erfindung, als sie antimikrobielle Eigenschaften aufweisen. Antimikrobielle Eigenschaften des Fragmentes bedeutet, dass das Fragment mindestens 50 % der Aktivität des Stammpeptides hat, also maximal die doppelte Hemmkonzentration benötigt. Dabei werden als Testmikroorganismen entweder Staphylococcus aureus oder Escherichia coli verwendet, je nachdem, bei welchem Mikroorganismus das Peptid bessere Wirksamkeit hat. Die Bakterien sind wie folgt charakterisiert:
Staphylococcous aureus: Gram-positive Kokken, ATCC 25923
Escherichia coll: Gram-negative Stäbchen, ATCC 25922

Die minimale Inhibitionskonzentration wurde in Anlehnung an die von dem NCCLS (National Committee for Clinical Laboratory Standards) herausgegebenen Empfehlungen (M7-A3) analog Beispiel 4 bestimmt.

Die erfindungsgemäßen Peptide können durch Isolierung aus menschlichen und tierischen Gewebeextrakten, durch Proteolyse aus korrespondierenden Vorläuferproteinen, durch chemische und biotechnologische Synthese in reiner und biologisch aktiver Form hergestellt werden.

Bei dem erfindungsgemäßen Peptid handelt es sich um ein Fragment von einem größeren Vorläuferprotein, nämlich mitochondrialem Proteolipid.

Die erfindungsgemäßen Peptide eignen sich zur Behandlung von durch mikrobielle Fehlbesiedlungen bedingten Erkrankungen wie Infektionen, Entzündungen, mikrobiell induzierten Tumoren, mikrobiell bedingten degenerativen Erkrankungen, Durchfallerkrankungen, Koliken, Abweichungen der Mund-, Darm- und Vaginalflora, Karies. Die mikrobielle Fehlbesiedlung kann beispielsweise durch Bakterien, Pilze, Hefen, Protisten, Viren, Mycoplasmen, Filarien und/oder Plasmodien bedingt sein. Sie können sowohl bei akuten als auch bei chronischen Erkrankungen eingesetzt werden.

Die Arzneimittelzubereitung enthält die erfindungsgemäßen Peptide bzw. ein physiologisch verträgliches Salz der Peptide. Die Form und Zusammensetzung des Arzneimittels, welches die Peptide enthält, richtet sich nach der Art der Verabreichung. Bevorzugt werden galenische Zubereitungen eingesetzt und Applikationsformen gewählt, bei denen die Peptide undegradiert an den Wirkort gelangen. Arzneimittelzubereitungen können pharmazeutisch übliche Hilfsstoffe, die z. B. einen Beitrag zur Löslichkeit, Stabilität oder Sterilität des Arzneimittels leisten oder den Wirkungsgrad der Aufnahme in den Körper erhöhen, enthalten. Die Peptide können parenteral, intranasal, oral und mittels Inhalation verabreicht werden. Vorzugsweise werden die Peptide zu einem Injektionspräparat, entweder als Lösung oder als Lyophilisat zur Auflösung unmittelbar vor Gebrauch, konfektioniert.

Die zu verabreichende Dosis der Peptide hängt von der Indikation und der Anwendung bestimmter Derivate ab. Vorzugsweise werden die erfindungsgemäßen Peptide in Mengen von 0,1 bis 100 mg/kg Körpergewicht eingesetzt.

Die erfindungsgemäßen Peptide können auch als Zusatzstoffe für Lebensmittel verwendet werden. Sie können bei der Herstellung von Lebensmitteln als Hilfsstoffe, insbesondere bei Lebensmitteln, die durch Gärung und andere bakterielle Prozesse hergestellt werden, verwendet werden. Bei den erfindungsgemäßen Präparaten handelt es sich um natürliche Konservierungsmittel.

Die folgenden Beispiele sollen die Erfindung weiter erläutern, wobei nur die Beispiele erfindungsgemäß sind, die das Peptid 5 der Seq ID 5 betreffen. Die übrigen Beispiele dienen als Vergleichsbeispiele.

### Beispiel 1

### Herstellung des Plazentaextrakts

Humane Plazenta wurde mit Extraktionspuffer (1,0 M Essigsäure, 20 mM Ascorbinsäure, 1 mM EDTA, pH 2,45) und Eis homogenisiert und zur Extraktion über Nacht bei 4 °C gerührt. Das extrahierte Homogenisat wurde zentrifugiert und der Überstand filtriert. Das erhaltene Filtrat wurde ultrafiltriert (50 kDa PS-Membran) und das Permeat nachfolgend auf einen Kationenaustauscher gegeben und stufenweise pH-Wert abhängig eluiert. In den Eluaten enthaltene Peptide/Proteine wurden über RP-Chromatographie weiter aufgetrennt.

### HPLC Reinigung von antimikrobiellen Peptiden aus Plazentaextrakt

Für die Reinigung von antimikrobiellen Peptiden aus Plazentaextrakt können verschiedene HPLC Trennverfahren kombiniert werden, um eine möglichst reine Darstellung über eine optimale Trennung zu erzielen und inaktive, unerwünschte Bestandteile abzutrennen. Die jeweiligen Proben, die nach jedem Trennungsschritt entstehen, werden auf ihre antimikrobielle Aktivität gegen *Escherichla coli* mit Hilfe des Radial-DifFusions-Hemmtests untersucht (siehe Beispiel 3). Notwendig für die Reinigung ist die Kombination von mindestens zwei Reverse Phase Chromatographieschritten und der Einsatz einer Kationenaustausch-HPLC-Trennung. In dem Biotest sollte die jeweilige Probe salzarm eingesetzt werden, um ein möglichst optimales Screeningergebnis zu erhalten.

Der erste Trennschritt wurde mit Hilfe einer Fineline RPC Polymer Säule (20 x 15,5 cm, 15 µm, Pharmacia Biotech, Freiburg, Deutschland) durchgeführt.

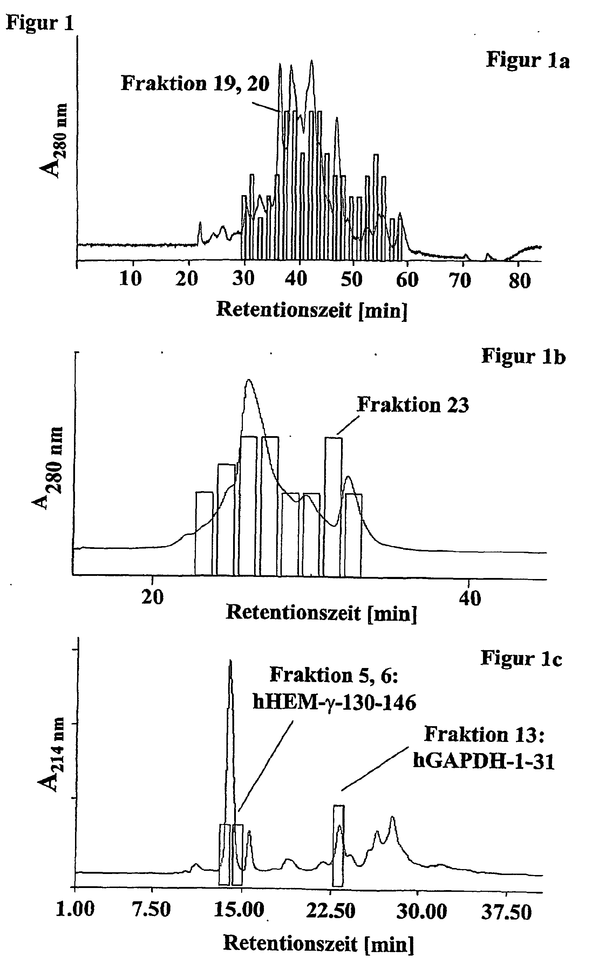

Puffer A: 10 mM HCl; Puffer B: 10 mM HCl / 80 % Acetonitril / 20 % bidestilliertes Wasser; Gradient: 0 -50 % B in 60 Minuten; Fluss: 400 ml/min; Detektion: 280 nm
Das Chromatogramm ist in Figur 1a dargestellt. Die Balken geben die antimikrobielle Aktivität der erhaltenen Fraktionen an.

Rechromatographie von Fraktion 19 und 20 mit einer YMC Gel C8 - C18 Säule (47 x 300 mm, 15 µm, 300 Å, Waters, Milford, MA).
Puffer A: 10 mM HCl; Puffer B: 10 mM HCl / 80 % Acetonitril / 20 % bidestilliertes Wasser; Gradient: 20 - 70 % B in 37,5 Minuten; Fluss: 40 ml/min; Detektion: 280 nm
Das Chromatogramm ist in Figur 1b dargestellt. Die Balken geben die antimikrobielle Aktivität der erhaltenen Fraktionen an.

Rechromatographie von Fraktion 23 des vorangegangenen Trennschrittes mit einer Kationenaustausch HPLC.
Säule: Parcosil Pepkat, 4 x 50 mm, 5 µm, 300 Å, Biotek, Heidelberg, Deutschland
Puffer A: 10 mM Natriumphosphatpuffer pH 7,2; Puffer B: Puffer A mit 1 M NaCl
Gradient: 0 - 60 % B in 60 Minuten; Fluss: 0,75 ml/min; Detektion: 214 nm.

Die erhaltenen Fraktionen wurden einzeln mit einem Reverse-Phase Chromatographieschritt entsalzt, bevor sie dem Test auf antimikrobielle Aktivität zugeführt wurden. Das Chromatogramm ist in Figur 1c dargestellt. Die Balken geben die antimikrobielle Aktivität der erhaltenen Fraktionen an.

Durch Massenspektrometrie und Aminosäuresequenzierung wurden die folgenden Peptide identifiziert:
Fraktion 5 + 6 enthielten das reine, antimikrobiell wirksame Peptid hHEM-g-130-146 mit der Aminosäuresequenz: WQKMVTAVASALSSRYH Seq ID No. 1
Fraktion 13 enthielt das reine, antimikrobiell wirksame Peptid hGAPDH-1-31 mit der Aminosäuresequenz: GKVKVGVNGFGRIGRLVTRAAFNSGKVDIVA Seq ID No. 2

### Beispiel 2

### Herstellung des Thymusextrakts

Kalbsthymus wurde mit Extraktionspuffer (1,0 M Essigsäure, 10 mM Ascorbinsäure, 0,5 mM EDTA) und Eis homogenisiert und zur Extraktion über Nacht bei 4 °C gerührt. Das extrahierte Homogenisat wurde zentrifugiert und der Überstand fltriert. Das erhaltene Filtrat wurde ultrafiltriert (50 kDa PS-Membran) und das Permeat nachfolgend auf einen Kationenaustauscher gegeben und stufenweise pH-Wert abhängig eluiert. In den Eluaten enthaltene Peptide/Proteine wurden über RP-Chromatographie weiter aufgetrennt.

### HPLC Reinigung von antimikrobiellen Peptiden aus Thymusextrakt

Für die Reinigung von antimikrobiellen Peptiden aus Thymusextrakt können verschiedene HPLC Trennverfahren kombiniert werden, um eine möglichst reine Darstellung über eine optimale Trennung zu erzielen und inaktive, unerwünschte Bestandteile abzutrennen. Die jeweiligen Proben, die nach jedem Trennungsschritt entstehen, werden auf ihre antimikrobielle Aktivität gegen *Escherichia coli* mit Hilfe des Radial-Diffusions-Hemmtests untersucht (siehe Beispiel 2). Empfehlenswert für die Reinigung ist die Kombination von mindestens zwei Reverse Phase Chromatographieschritten und der Einsatz einer Kationenaustausch-HPLC-Trennung. In dem Biotest sollte die jeweilige Probe salzarm eingesetzt werden, um ein möglichst optimales Screeningergebnis zu erhalten.
Der erste Trennschritt wurde mit Hilfe einer Reverse Phase RP C15 Säule (10 x 15 cm, 15 µm, Pharmacia Biotech, Freiburg, Deutschland) durchgeführt.Puffer A: 10 mM HCl; Puffer B: 10 mM HCl / 80 % Acetonitril / 20 % bidestilliertes Wasser; Gradient: 1 % B / min, 60 Minuten; Fluss: 130 ml/min; Detektion: 280 nm

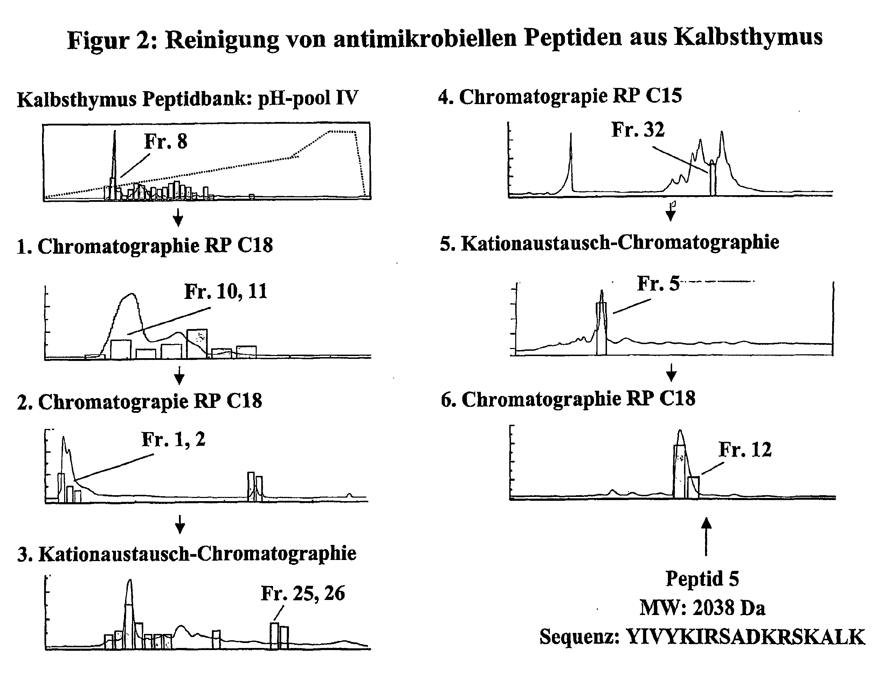

Das Chromatogramm ist In Figur 2 (pH-pool IV) dargestellt. Die Balken geben die antimikrobielle Aktivität der erhaltenen Fraktionen an.

Rechromatographie von Fraktion 8 mit einer YMC C18 Säule (47 x 300 mm, 15 µm, 300 Å, Waters, Milford, MA, USA). Puffer A: 10 mM HCl; Puffer B: 10 mM HCl / 80 % Acetonitril / 20 % bidestilliertes Wasser; Gradient: 10 - 60 % B in 50 min; Fluß: 40 ml/min; Detektion: 280 nm Das Chromatogramm ist in Figur 2 (1. Chromatographie) dargestellt. Die Balken geben die antimikrobielle Aktivität der erhaltenen Fraktionen an.
Rechromatographie von Fraktionen 10 und 11 mit einer YMC C18 Säule (47 x 300 mm, 15 µm, 300 Å, Waters, Milford, MA, USA). Puffer A: 20 % Methanol in 10 mM HCl; Puffer B: Methanol in 10 mM HCl; Gradient: 10 - 50 % B in 57 min; Fluß: 30 ml/min; Detektion: 280 nm Das Chromatogramm ist in Figur 2 (2. Chromatographie) dargestellt. Die Balken geben die antimikrobielle Aktivität der erhaltenen Fraktionen an.

Rechromatographie von Fraktionen 1 und 2 des vorangegangenen Trennschrittes mit Hilfe eines Kationenaustauschers.
Säule: Biotek Prepkat, 10 x 125 mm, 7 µm, 1000 Å, Biotek, Heidelberg, Deutschland
Puffer A: 50 mM Kaliumdihydrogenphosphatpuffer, pH 3,0; Puffer B: Puffer A mit 1,5 M KCI
Gradient: 10 - 60 % B in 50 min; Fluss: 2 ml/min; Detektion: 230 nm
Das Chromatogramm ist in Figur 2 (3. Chromatographie) dargestellt. Die erhaltenen Fraktionen wurden einzeln mit einem Reverse-Phase Chromatographieschritt entsalzt, bevor sie dem Test auf antimikrobielle Aktivität zugeführt wurden. Die Balken geben die antimikrobielle Aktivität der erhaltenen Fraktionen an.

Rechromatographie von Fraktionen 25 und 26 mit einer RP C15 Säule (10 x 250 mm, 15 µm, 120 Å, Pharmacia Biotech, Freiburg, Deutschland). Puffer A: 0,1 % TFA; Puffer B: 80 % Acetonitril in 0,085 % TFA; Gradient: 20 - 50 % B in 60 min; Fluß: 1,8 ml/min; Detektion: 214 nm Das Chromatogramm ist in Figur 2 (4. Chromatographie) dargestellt. Die Balken geben die antimikrobielle Aktivität der erhaltenen Fraktionen an.

Rechromatographie von Fraktion 32 des vorangegangenen Trennschrittes mit Hilfe eines Kationenaustauschers.
Säule: Parcosil Pepkat, 4 x 50 mm, 5 µm, 300 Å, Biotek, Heidelberg, Deutschland
Puffer A: 10 mM Natriumphosphatpuffer pH 7,2; Puffer B: Puffer A mit 1 M NaCl
Gradient: 0 - 60 % B in 60 Minuten; Fluss: 0,75 ml/min; Detektion: 214 nm.

Die erhaltenen Fraktionen wurden einzeln mit einem Reverse-Phase Chromatographieschritt entsalzt, bevor sie dem Test auf antimikrobielle Aktivität zugeführt wurden. Das Chromatogramm ist in Figur 2 (5. Chromatographie) dargestellt. Die Balken geben die antimikrobielle Aktivität der erhaltenen Fraktionen an.
Rechromatographie von Fraktion 5 mit einer Vydac RP C18 Säule (4,6 x 250 mm, 5 µm, 100 Å, Hesperia, CA, USA). Puffer A: 0,1 % TFA; Puffer B: 80 % Acetonitril in 0,085 % TFA; Gradient: 20 - 50 % B in 60 min; Fluss: 0,75 ml/min; Detektion: 214 nm Das Chromatogramm ist in Figur 2 (6. Chromatographie) dargestellt. Die Balken geben die antimikrobielle Aktivität der erhaltenen Fraktionen an.
Durch Massenspektrometrie und Aminosäuresequenzierung wurde das folgende Peptid identifiziert:
Fraktion 12 enthielt das reine, antimikrobiell wirksame Peptid 5 mit der Aminosäuresequenz: YIVYKIRSADKRSKALK Seq ID No. 5

### Beispiel 3

### Nachweis der antimikrobiellen Aktivität auf E. coli

Beim Radial-Diffusions-Hemmtest wird die antimikrobielle Wirkung von Probelösungen, hier HPLC Fraktionen, auf in Agarose suspendierte Bakterien, hier *E. coli,* untersucht. Dazu wurden die HPLC-Fraktionen lyophilisiert und in bidestilliertem Wasser aufgenommen. Zum Test ihrer antimikrobiellen Aktivität wurde wie folgt verfahren: zu dem bei 37 °C inkubierten Medium (300 mg/l Tryptic Soy Broth, 0,8 % (w/v) NuSieve GTG Agarose, 0,02 % (v/v) Tween 20 in 10 mM Natriumphosphatpuffer pH 7,2) wurde eine Bakteriensuspension (in LB-Medium), die eine optische Dichte von 1,0 bei einer Wellenlänge von 600 nm aufwies, gegeben. Nach Abkühlen der Agaroselösung in Petrischalen und Aushärtung bei 4 °C wurden steril Kavitäten mit einem Durchmesser von 3 mm in die feste Agarose gestanzt. In die Kavitäten wurden 10 µl Probelösung gegeben und die bestückten Platten bei 37 °C für 16 h inkubiert. Zur Auswertung der antimikrobiellen Aktivität der Proben wurden die entstandenen Hemmhöfe vermessen. Es besteht eine lineare Korrelation zwischen dem Logarithmus der Antibiotikakonzentration und den Hemmhofradien. Zum Vergleich antimikrobieller Aktivität verschiedener Fraktionen wurden entsprechend der Arbeit von Lehrer (Lehrer et al., J Immun Methods, Vol 137, S. 167, 1991) die Hemmhofradien in Einheiten umgerechnet. Dabei entspricht 1 Unit einem Hofradius von 0,1 mm.

### Beispiel 4

### Antimikrobielle Wirkung der erfindungsgemäßen Peptide gegen Bakterien und Hefen

Die antimikrobielle Aktivität der erfindungsgemäßen Peptide wurde unter Anwendung der Bestimmung der minimalen Hemmstoffkonzentration (MIC) gegen verschiedene Keime, wie Gram-positive und Gram-negative Bakterien und Hefen, untersucht. Die minimale Hemmstoffkonzentration bezeichnet die Peptidkonzentration, die minimal erforderlich ist, um das Wachstum von Mikroorganismen nach einer Inkubationszeit von 18 ± 2 h vollständig zu hemmen. Diese Quantifizierung der antimikrobiellen Aktivität ist mit chemisch synthetisierten Peptiden (Peptidsequenzen 1-5) gegen nicht pathogene sowie human pathogene Erreger durchgeführt worden. Die minimalen Hemmkonzentrationen sind in der nachfolgenden Tabelle in [µg/ml] angegeben:

| Peptide | 1 | 2 | 3 | 4 | 5 | 6 | 7 | 8 | 10 |
|---|---|---|---|---|---|---|---|---|---|
| Bacillus subtilis ATCC 6051 | 100 | 7.8 | 62.5 | 62.5 | 1.95 | 25 | 3,9 | nd | 20 |
| Micrococcus luteus ATCC 9341 | 200 | 31.25 | 31.25 | 200 | 6.25 | 50 | 25 | 31.2 5 | 18.7 5 |
| Staphylococcus aureus ATTC 25923 | 200 | nd | nd | > 200 | 25 | 100 | 25 | 100 | 200 |
| Staphylococcus carnosus TM 300 | 100 | 12.5 | 40 | 50 | 6.25 | 25 | 15,6 | 12.5 | 37.5 |
| Escherichia coli ATTC 25922 | 200 | nd | nd | >200 | 6.25 | 50 | 12,5 | >20 0 | 100 |
| Escherichia coli BL21 (DE3) | 100 | 7.8 | 62.5 | 100 | 1.95 | 6,2 | 3,9 | >25 0 | 9.4 |
| Saccharomyces cerevisiae ATCC 9763 | 100 | 100 | 50 | 100 | 3.125 | 31,3 | 25 | >25 0 | 25 |
| Salmonella typhimurium ATCC 13311 | nd | nd | nd | nd | 10 | nd | nd | nd | nd |
| Pseudomonas aeruginosa clinical isolate | > 200 | 31.25 | > 200 | nd | 62.5 | nd | nd | nd | nd |
| Klebsiella pneumoniae ATCC 10031 | 300 | nd | nd | >300 | 150 | 75 | 18,75 | 100 | 25 |
| Mycobacterium ranae ATCC 110 | nd | nd | nd | nd | 30 | nd | nd | nd | nd |

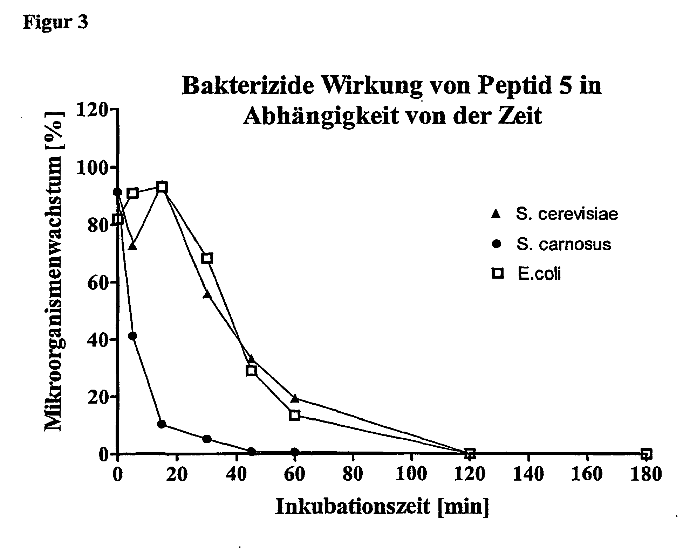

Die erfindungsgemäßen Peptide inhibieren effektiv sowohl das Wachstum von Gram-positiven und Gram-negativen Bakterien als auch von Hefen. Human pathogene Erreger, wie *Pseudomonas aeruginosa, Salmonella typhimurium* und *Klebsiella pneumoniae* werden ebenfalls effektiv abgetötet. Versuche zur Wirkkinetik ergaben, dass eine vollständige Abtötung der Keime bereits nach 2 h Inkubation erfolgte (siehe Figur 3).

## Patentansprüche

1. Peptid mit der Aminosäuresequenz sowie
- die amidierten, acetylierten, sulfatierten, phosphorylierten, glycosylierten, oxydierten Derivate,
- Peptide, die durch konservativen Austausch von Aminosäuren erhalten wurden,
- Peptide die durch Deletionen von nicht mehr als 10% der Aminosäuren des Peptides der Seq. ID No. 5 erhalten wurden und
- Fragmente des Peptids der Seq. ID No. 5
mit der Maßgabe, dass die Derivate, Fragmente und Peptide eine antimikrobielle Wirkung von mindestens 50% der antimikrobiellen Wirkung des Peptids mit Seq. ID No. 5 aufweisen.

2. Verfahren zur Herstellung der Peptide gemäß Anspruch 1 durch Aufreinigung aus menschlichen oder tierischen Gewebeextrakten oder aus menschlichen oder tierischen Körperflüssigkeiten, durch Proteolyse aus korrespondierenden Vorläuferproteinen, durch Expression in prokaryontischen oder eukaryontischen Organismen und durch chemische Synthese.

3. Arzneimittel, enthaltend mindestens ein Peptid gemäß Anspruch 1 als aktiven Wirkstoff zusammen mit pharmazeutisch üblichen Hilfs- und Zusatzstoffen.

4. Nahrungsmittel, enthaltend Peptide gemäß Anspruch 1 zusammen mit Nähr- und Zusatzstoffen.

5. Verwendung der Peptide gemäß Anspruch 1 in reiner Form zur Herstellung eines antibiotischen Pharmakons zur Behandlung von mikrobiell ausgelösten Erkrankungen.

6. Verwendung der Peptide nach Anspruch 5 in geeigneter Form, zubereitet für Infusionen, Salben, Tabletten, Sprays, "slow release"-Kapseln und ähnlichen Präparationen.

7. Verwendung der Peptide gemäß Anspruch 1 zur Herstellung eines Arzneimittels zur Behandlung von Erkrankungen des menschlichen Organismus mit Beteiligung des Magen-Darm-Traktes, der Atemwege und des Urogenitaltraktes, und/oder zur Behandlung von Erkrankungen des menschlichen Organismus mit Beteiligung des Integumentes und seiner Anhangsdrüsen.

8. Verwendung der Peptide gemäß Anspruch 1 zur Herstellung eines Arzneimittels zur Behandlung von durch mikrobielle Fehlbesiedlung bedingten Erkrankungen, verursacht durch Bakterien, Pilze, Hefen, Protisten, Viren, Mycoplasmen, Filarien und/oder Plasmodien.

9. Verwendung nach Anspruch 8, **dadurch gekennzeichnet, dass** die mikrobiellen Fehlbesiedlungen Infektionen, Entzündungen, mikrobiell induzierte Tumore, mikrobiell bedingte degenerative Erkrankungen, Durchfallerkrankungen, Koliken, Abweichungen der Mund-, Darm- und Vaginalflora und/oder Karies sind.

10. Verwendung der Peptide gemäß Anspruch 1 als Konservierungsstoff von Lebens- und Futtermitteln und/oder als Hilfsstoff bei industriellen Gärprozessen, insbesondere bei der Bierherstellung, bei der Joghurtproduktion usw., um die vorliegende Bakterienflora zu modifizieren.

## Claims

1. A peptide having the amino acid sequence and
- its amidated, acetylated, sulfated, phosphorylated, glycosylated, oxidized derivatives;
- peptides obtained therefrom by a conservative substitution of amino acids;
- peptides obtained by deletions of not more than 10% of the amino acids of the peptide of SEQ ID No. 5; and
- fragments of the peptide of SEQ ID No. 5;
with the proviso that said derivatives, fragments and peptides have an antimicrobial activity which is at least 50% of the antimicrobial activity of the peptide of SEQ ID No. 5.

2. A process for preparing the peptides according to claim 1 by purification from human or animal tissue extracts or from human or animal body fluids, by proteolysis from corresponding precursor proteins, by expression in prokaryotic or eukaryotic organisms, and by chemical synthesis.

3. A medicament containing at least one peptide according to claim 1 as an active ingredient together with pharmaceutically acceptable auxiliary agents and additives.

4. A foodstuff containing peptides according to claim 1 together with nutrients and additives.

5. Use of the peptides according to claim 1 in a pure form for preparing an antibiotic medicament for the treatment of microbially caused diseases.

6. Use of the peptides according to claim 5 in a suitable form, formulated for infusions, ointments, tablets, sprays, "slow release" capsules and similar preparations.

7. Use of the peptides according to claim 1 for preparing a medicament for the treatment of diseases of the human organism involving the gastro-intestinal tract, the respiratory tract and the urogenital tract, and/or for the treatment of diseases of the human organism involving the integument and its appendage glands.

8. Use of the peptides according to claim 1 for preparing a medicament for the treatment of diseases caused by misplaced microbial colonizations caused by bacteria, fungi, yeasts, protists, viruses, mycoplasmas, filariae and/or plasmodiums,

9. The use according to claim 8, **characterized in that** said misplaced microbial colonizations are infections, inflammations, microbially induced tumors, microbially caused degenerative diseases, diarrheic diseases, colics, deviations in the oral, intestinal and vaginal floras, and/or caries.

10. Use of the peptides according to claim 1 as a preservative for foodstuffs and fodders and/or as an auxiliary agent in industrial fermentation processes, especially in beer production, yoghurt production etc, for modifying the existing bacterial flora.

## Revendications

1. Peptides ayant la séquence d'acide aminé suivante : ainsi que:
- les dérivés amidifiés, acétylisés, sulfatés, phosphorylés, glycosylés, oxydés,
- les peptides qui sont obtenus par substitution conservative d'acides aminés,
- les peptides qui sont obtenus par délétion de pas plus de 10% des acides aminés du peptide de la séquence Seq. ID No.5, et
- les fragments du peptide de la séquence Seq. ID No.5,
pourvu que les dérivés, fragments et peptides présentent une activité antimicrobienne d'au moins 50% de l'activité antimicrobienne du peptide avec la séquence Seq. ID No.5

2. Procédé de préparation de peptides selon la revendication 1 par purification d'extraits de tissus humains ou animaux ou de fluides corporels humains ou animaux, par protéolyse de protéines précurseurs correspondantes, par expression dans des organismes procaryotes ou eucaryotes et par synthèse chimique.

3. Médicament contenant au moins un peptide selon la revendication 1 en tant que principe actif mélangé à des additifs ou à des ingrédients pharmaceutiques habituels.

4. Denrée alimentaire contenant un peptide selon la revendication 1 mélangé à des denrées alimentaires et des additifs.

5. Utilisation des peptides selon la revendication 1 sous forme pure pour la préparation d'un principe actif antibiotique pour le traitement des maladies d'origine microbienne.

6. Utilisation des peptides selon la revendication 5 sous une forme appropriée, préparée pour des infusions, des pommades, des comprimés, des aérosols, des capsules à libération lente et des préparations semblables.

7. Utilisation des peptides selon la revendication 1 pour la préparation d'un médicament destiné au traitement des maladies de l'organisme humain dans lesquelles sont impliqués le système gastro-intestinal, les voies respiratoires et le système urogénital, et/ou destiné au traitement des maladies de l'organisme humain dans lesquelles sont impliqués les phanères et leurs glandes associées.

8. Utilisation d'un peptide selon la revendication 1 pour la préparation d'un médicament destiné au traitement de maladies provoquées par une mauvaise colonisation microbienne, provoquées par des bactéries, des champignons, des levures, des protistes, des virus, des mycoplasmes, des filaires et/ou des plasmodies.

9. Utilisation selon la revendication 8, **caractérisée en ce que** les mauvaises colonisations microbiennes sont des infections, des inflammations, des tumeurs induites par des microbes, des maladies dégénérescentes déclenchées par voie microbienne, des diarrhées, des coliques, des aberrations de la flore buccale, intestinale ou vaginale ou des caries.

10. Utilisation des peptides selon la revendication 1 en tant qu'agent de conservation des denrées alimentaires et des aliments pour animaux et/ou en tant qu'additif dans les procédés industriels de fermentation, notamment pour la fabrication de la bière, la production de yaourts, etc...afin de modifier la flore bactérienne existante.


### SEQUENZ PROTOKOLL

<210> 5
   <211> 17
   <212> PRT
   <213> mammalian
<400> 5
   Tyr Ile Val Tyr Lys Ile Arg Ser Ala Asp Lys Arg Ser Lys Ala Leu
    1               5                  10                  15
   Lys